# EUROPEAN PATENT APPLICATION

(11) **EP 3 747 483 A1**
(43) Date of publication of application: **09.12.2020**
(21) Application number: 20178468.3
(22) Date of filing: 05.06.2020
(51) Int. Cl.: A61M 3/02, A61M 35/00

(54) **WOUND IRRIGATION DEVICE**

(30) Priority: 06.06.2019 US 201916433276
(71) Applicant: Orlando Health, Inc., Orlando, FL 32806 (US)
(72) Inventor: Rosenberg, Marcy Sara, Orlando, FL Florida 32803 (US); Solar, Mathew S., Indialantic, FL Florida 32903 (US)
(74) Representative: Zacco Denmark A/S

(57) **Abstract**

A wound irrigation device may include a barrel having a fluid chamber at least partially filled with an irrigation fluid. A piston may be located in the barrel, where the piston is movable between a terminal proximal position and a terminal distal position within the barrel to control a volume of the fluid chamber of the barrel. An actuation spring may be mechanically coupled to the piston, where the actuation spring is configured such that a pressure of the irrigation fluid within the fluid chamber continuously exceeds about 4 psi as the piston moves between the terminal proximal position and the terminal distal position.

## Description

### TECHNICAL FIELD

The present disclosure is generally related to a wound irrigation device designed to promote wound healing. More particularly, the present disclosure describes a wound irrigation device that provides a stream of pressurized irrigation fluid and that has a compact, portable design.

### BACKGROUND

Wounds to human and animal bodies, whether formed through accident/injury or intentionally (e.g., through a surgical procedure), are typically cleansed in a medical setting to remove bacteria and other matter that increase the risk of infection and/or slow the rate of healing. One method of cleansing a wound involves spraying the wound with a stream of pressurized fluid (e.g., typically a sterile liquid, such as a saline solution) to remove foreign particles.

While increased pressure typically corresponds with an increased effectiveness of removing bacteria and other undesirable matter, stream under too much pressure may cause additional injury to the wound by damaging the wound's regenerating tissue. A relatively narrow pressure range exists where the stream of irrigation fluid is effective against foreign particles, yet causes minimal damage to healing tissue. For example, present guidelines call for a pressure of between about 8 psi and about 12 psi (at least for certain wound types).

When using known wound irrigation devices, the irrigation fluid is typically delivered to a target site (within a wound) by actuating a hypodermic syringe and directing the resulting stream of irrigation fluid towards the target site by handcontrolling the fluid discharge stream (e.g., by manually changing the direction that the syringe barrel is facing). While such devices have been used with some success and do not require an external actuation source (such that they can be used in a variety of locations), they have certain shortcomings. For example, control of the fluid pressure may be difficult to maintain within the acceptable range, particularly while the medical professional (or other operator) is focused on aiming the stream of irrigation fluid at a target site.

In light of this background, the present disclosure is related to a wound irrigation device that provides a stream of irrigation fluid within an acceptable pressure range while also having a compact, portable design.

### BRIEF SUMMARY

One aspect of the present disclosure includes a wound irrigation device. The wound irrigation device may include a barrel having a fluid chamber at least partially filled with an irrigation fluid, a piston located in the barrel, where the piston is movable between a terminal proximal position and a terminal distal position within the barrel to control a volume of the fluid chamber of the barrel, and an actuation spring that is mechanically coupled to the piston. The actuation spring may be configured such that a pressure of the irrigation fluid within the fluid chamber continuously exceeds about 4psi as the piston moves between the terminal proximal position and the terminal distal position.

Another general aspect includes a wound irrigation device with a barrel having a fluid chamber configured to be at least partially filled with an irrigation fluid, a piston located in the barrel, where the piston is movable within the barrel to control a volume of the fluid chamber of the barrel, and an actuation spring mechanically coupled to the piston. During an operational state, the actuation spring applies a distal actuation force on the piston to pressurize the irrigation fluid within the fluid chamber.

Another general aspect includes a wound irrigation device with a barrel having a fluid chamber and a piston located in the barrel, where the piston is movable within the barrel to control a volume of the fluid chamber of the barrel. The wound irrigation device may include a locked state and an operational state, where in the locked state, the piston is fixed relative to the barrel, and where in the operational state, an actuation force applied to the piston in the distal direction pressurizes an irrigation fluid within the fluid chamber.

### BRIEF DESCRIPTION OF THE DRAWINGS

The embodiments of the present disclosure may be better understood with reference to the following drawings and description. The components in the figures are not necessarily to scale, with emphasis instead being placed upon illustrating the principles of the present disclosure. Moreover, in the figures, like referenced numerals designate similar or identical features.
FIG. 1 is an illustration showing a wound irrigation device having a piston in a first position in accordance with certain aspects of the present disclosure.
FIG. 2 is an illustration showing the wound irrigation device having the piston in a second position in accordance with certain aspects of the present disclosure.
FIG. 3 is an illustration showing another embodiment of a wound irrigation device having a conically-tapered compression spring in accordance with certain aspects of the present disclosure.
FIG. 4 is an illustration showing another embodiment of a wound irrigation device having an extension spring in accordance with certain aspects of the present disclosure.
FIG. 5 is an illustration showing another embodiment of a wound irrigation device having a pressure source chamber in accordance with certain aspects of the present disclosure.
FIG. 6 is an illustration showing a housing and a trigger for a wound irrigation device in accordance with certain aspects of the present disclosure.
FIG. 7 is an illustration showing a portion of the housing depicted in FIG. 5 along with certain other components of a wound irrigation device in accordance with certain aspects of the present disclosure.
FIG. 8 is an illustration showing another embodiment of a wound irrigation device where an actuation spring acts directly on a piston in accordance with certain aspects of the present disclosure.
FIG. 9 is an illustration showing a removable nozzle assembled or formed with a splash shield in accordance with certain aspects of the present disclosure.
FIGS. 10A-D are illustrations showing a set of potential nozzle designs for use with a wound irrigation device in accordance with certain aspects of the present disclosure.

### DETAILED DESCRIPTION

Various aspects are described below with reference to the drawings in which like elements generally are identified by like numerals. The relationship and functioning of the various elements of the aspects may be better understood by reference to the following detailed description. However, aspects are not limited to those illustrated in the drawings or explicitly described below. It also should be understood that the drawings are not necessarily to scale, and in certain instances details may have been omitted that are not necessary for an understanding of aspects disclosed herein, such as conventional fabrication and assembly.

The terms "distal" and "proximal" are used herein in the common usage sense where they refer respectively to a tool/patient-end of a device or related object and the opposite end (e.g., typically the handle/doctor-end of the device or related object).

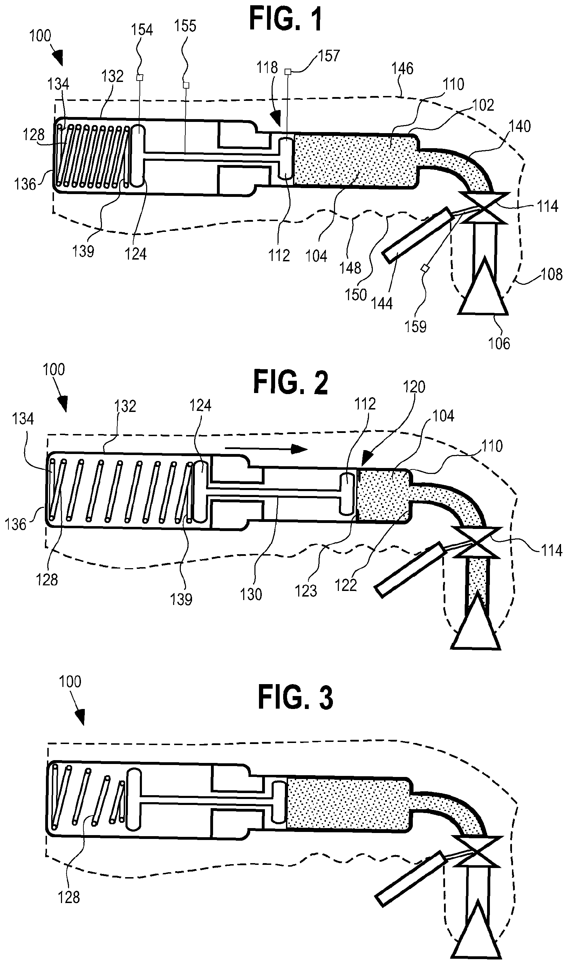

FIG. 1 shows an example of a wound irrigation device 100 in accordance with certain aspects of the present disclosure. As shown, the wound irrigation device 100 may generally include a barrel 102 having a fluid chamber 104 that selectively communicates with an irrigation outlet 106 located at a distal end 108 of the device (which may include a nozzle 162, as discussed in more detail below). Without limitation, the fluid chamber 104 may be generally cylindrical and may be defined by inner walls of the barrel 102, though other shapes may also be used. For example, the barrel 102 may be similar (or identical) to a barrel of a syringe.

Prior to and during a wound irrigation procedure, the fluid chamber 104 of the barrel 102 may be at least partially filled with an irrigation fluid 110, such as a liquid and/or other fluid (e.g., a compressible gas) that is suitable for directing towards a wound in a pressurized stream. Suitable irrigation fluids include, but are not limited to, liquids such as aqueous topical antibiotic solutions, isotonic saline, or the like.

A piston 112 may be located in the barrel 102 and configured to provide a distal-direction force for pressurizing the irrigation fluid 110 in the fluid chamber 104. This pressure may cause the irrigation fluid 110 to discharge through the irrigation outlet 106 (e.g., when a valve 114 is open). Further, the piston 112 may be movable within the barrel 102 to continuously provide pressure as the irrigation fluid 110 is discharged.

At the initiation of an irrigation procedure, the piston 112 may be located at a first position 118 (shown in FIG. 1). In some embodiments, the first position 118 may be a terminal proximal position of the piston 112, which is defined herein as the proximalmost position that the piston 112 is located within the barrel 102 during normal operation (e.g., during a wound irrigation procedure). As the irrigation fluid 110 is discharged (and thus takes up less volume, at least when an incompressible fluid is used), the distal-direction force applied to the piston 112 may cause the piston 112 to move distally within the barrel 102, thus reducing the volume of the fluid chamber 104. The piston 112 may be movable at least from the first position 118 to a second position 120, shown in FIG. 2. Optionally, the second position 120 may be a terminal distal position of the piston 112, defined herein as the distal-most position that the piston 112 is located within the barrel 102 during normal operation (e.g., during a wound irrigation procedure). For example, the second position 120 may be a location where the piston 112 contacts a distal wall 122 of the barrel 102 (i.e., further distally than what is depicted in FIG. 2), but other structures may be used to prevent further distal movement of the piston 112 (e.g., the depicted stop/protrusion 123 that extends from the inner wall of the barrel 102 for contacting the piston 112, a wall or stop/protrusion that is engageable with the anchor 124, etc.).

The size of the barrel 102, and/or maximum displacement of the piston 112, may be specifically designed to accommodate a selected amount of irrigation fluid 110. For example, and without limitation, the barrel 102 and/or maximum displacement of the piston 112 may be configured such that the wound irrigation device 100 can discharge at least about 10 cubic centimeters (cc) of irrigation fluid 110, such as at least about 50 cc, at least about 100 cc, at least about 150 cc, at least about 200 cc, or more. During substantially the entirety of the discharge (i.e., containing at least 90% of the total discharge), the pressure of the irrigated stream may remain at or above a minimum pressure, such as at least about 4 pounds per square inch (psi), at least about 6 psi, at least about 8 psi, at least about 10 psi, or higher.

Any suitable actuation system or device may be used to apply the distal-direction force126 to the piston 112 (and therefore to pressurize the irrigation fluid 110 within the fluid chamber 104). For example, the wound irrigation device 100 may include an actuation spring 128 that is mechanically coupled to the actuation spring 128. The mechanical communication between the actuation spring 128 and the piston 112 in the depicted embodiment is provided through a second piston (or other suitable structure), herein referred to as the anchor 124, that is fixed to the piston 112 via a shaft 130. The shaft 130, which is an optional component, may be advantageous in embodiments where the piston 112 and the anchor 124 are distanced from one another (e.g., due to being located within chambers of difference sizes). In certain embodiments, the anchor 124 (and/or shaft 130) may be unnecessary (e.g., if the actuation spring 128 directly contacts the piston 112, for example).

The actuation spring 128 (and the anchor 124) may be contained in a spring chamber 132. As shown in FIGS. 1-2, a proximal end 134 of the actuation spring 128 may be fixed to a proximal wall 136 or other structure to base/fix the proximal end 134 of the actuation spring 128 relative to the barrel 102. A distal end 139 of the actuation spring 128 may be fixed to the anchor 124. As a result, when the actuation spring 128 is in a compressed state, the actuation spring 128 may act as a source of potential energy for applying the distal-direction force126 to the anchor 124 (and therefore to the piston 112) to cause pressure within the fluid chamber 104. The spring chamber 132 may be advantageous for providing protecting the actuation spring 128 from external objects, particularly since the actuation spring 128 may be stored in a compressed state (as discussed in more detail below).

The actuation spring 128 may have select characteristics for providing fluid pressure within a specified range that is desirable for wound irrigation. For example, the actuation spring 128 may have a particular spring constant and length (and/or other dimension) selected for providing a specific pressure range for the entirety of time that the piston 112 is between the first position 118 (of FIG. 1) and the second position 120 (of FIG. 2). In some embodiments, when the piston 112 is in the first position 118 (typically at the initiation of a wound irrigation procedure), the degree of compression of the actuation spring 128 may be such that the actuation spring 128 causes a fluid pressure of at least about 6 psi within the fluid chamber, such as at least about 8 psi, at least about 10 psi, at least about 12 psi, at least about 14 psi, or higher. In certain exemplary embodiments (and without limitation), the target pressure within the fluid chamber 104 (provided by the actuation spring 128 in an active/operational state with the valve 114 open) may be about 12 psi when the piston 112 is in the first position 118.

As the piston 112 moves towards the second position 120, the pressure within the fluid chamber 104 may drop proportionately or disproportionately due to release of potential energy from the actuation spring 128 (i.e., due to Hooke's Law, stating that the force provided by a compression spring is proportional to the distance that it is stretched/compressed). However, as it may be desirable to provide sufficient pressure for wound irrigation throughout the entire movement of the piston 112, the characteristics of the actuation spring 128 may be selected such that it is not completely exhausted of potential energy when the piston 112 reaches the second position 120, or other terminal proximal position where the irrigation fluid 110 is exhausted. For example, by using a spring with a pre-loaded rest position (i.e., the position of the spring at the end of its range of motion, when the piston 112 is in its terminal distal position) and a relatively long travel characteristic, the actuation spring 128 may be approximated as a "constant-force spring," or a spring for which the force it exerts over its range of motion is constant (which may be advantageous for providing approximately constant irrigation pressure). In practice, just prior to when the piston 112 reaches its terminal distal position (and is prevented from moving farther distally), the pressure within the fluid chamber 104 may be at least about 2 psi, such as at least about 4 psi, at least about 6 psi, at least about 8 psi, at least about 10 psi, or higher. In certain exemplary embodiments (and without limitation), the target pressure within the fluid chamber 104 (provided by the actuation spring 128 in an active/operational state with the valve 114 open) may be about 8 psi when the piston 112 is in the second position 120.

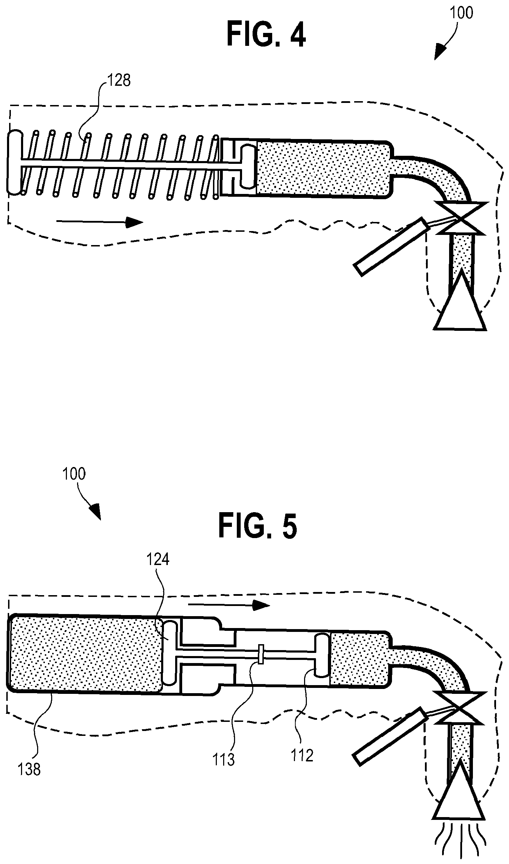

Any suitable spring type may be used for the actuation spring 128. As shown in FIGS. 1-2, for example, the actuation spring 128 may be a cylindrical compression spring that has a substantially-constant diameter along its length. As shown in FIG. 3, the actuation spring 128 may alternatively be a conically-tapered compression spring, which may be advantageous for providing narrower pressure range without increasing the spring size, and/or may have enhanced stability (from buckling or bending) relative to a spring having a constant diameter. Other spring types are also contemplated, such a rolled ribbon of spring steel, which may provide a good approximation of a constant-force spring. As shown in FIG. 4, the actuation spring 128 may be an extension spring rather than a compression spring, and therefore may be in a stretched state (relative to its stress-free default) during a wound irrigation procedure. Any other suitable spring type or combination of spring types may be used, and in some embodiments, a different source of potential energy may be used altogether. For example, referring to FIG. 5, the source of potential energy for moving the piston 112 may be provided by a pressurized source chamber 138 (e.g., filled with pressurized gas), which may be modular (e.g., allowing for selection of a particular pressure range by selection of a particular pressurized source chamber 138, etc.).

As mentioned above, fluid communication between the fluid chamber 104 and the irrigation outlet 106 may be controlled by a valve 114 (e.g., located in an optional curved flow path 140). During an irrigation procedure, a medical professional may operate the device by selectively controlling the valve 114. Accordingly, a trigger 144 or other suitable valve control interface (e.g., a button, knob, electronic control device, etc.) may be operatively coupled to the valve 114 such that the medical professional can quickly and efficiently influence the state of the valve 114. Further, the valve 114 may be only partially openable (e.g., via a selected degree of trigger movement) such that the pressure of the discharged fluid stream from the irrigation outlet 106 may be directly controlled by the operator. As shown, the trigger 144 may extend to a location outside of a housing 146 of the wound irrigation device 100 such that it is easily accessible. Optionally, the housing 146 may be sized and shaped to include an ergonomic handle 148 (e.g., with one or more finger depressions 150) for enhanced operator precision and comfort, and the trigger 144 may be located adjacent to the ergonomic handle 148 such that the medical professional or other operator can access and actuate the trigger 144 without substantial hand movement.

In some embodiments, the wound irrigation device 100 may include at least one locking device that is configured to engage at least one of the piston 112, the anchor 124, and/or another suitable structure (e.g., one that is fixed to the piston 112) such that, when in a locked state, the piston 112 relative to the barrel 102. For example, and as shown in FIG. 1, a first locking pin 154 may extend through the spring chamber 132 and engage the anchor 124 such that the anchor 124 (and therefore also the piston 112) is fixed in place. Additionally or alternatively, a second locking pin 155 may engage the shaft 130, and/or a third locking pin 157 may engage the piston 112. Advantageously, the locked state of the wound irrigation device 100 caused by engagement of the locking device152 may prevent the force caused by compression of the actuation spring 128 from being transformed into fluid pressure within the fluid chamber 104. When one or more locking devices is engaged, the pressure in the fluid chamber 104 may be less than about 8 psi, such as less than about 4 psi, or even less (e.g., nearly atmospheric pressure). By virtue of this reduced pressure, wear and tear on the device (e.g., on the valve 114), and/or unintentional activation of the device (e.g., via unintentional actuation of the trigger) prior to intentional initiation of a wound irrigation procedure. This feature may provide commercial feasibility for pre-filling the wound irrigation device 100, and/or pre-loading the actuation spring 128 (e.g., at a manufacturing facility and prior to shipment to a medical facility or patient home). For example, the locking device152 may be engaged during shipment and storage of the wound irrigation device 100 when the wound irrigation device 100 is manufactured and/or filled with the irrigation fluid 110.

Other suitable structures may alternatively (or additionally) be included to lock the piston 112 (relative to the barrel 102), such as a clip, bracket, clamp, screw, or the like. Further, as an additional (or alternative) measure, a second locking device may be included to prevent movement of the trigger 144 (or other actuator). In the depicted embodiment of FIG. 1, for example, the second locking device includes a second removable pin 159 that, when engaged with the trigger 144, prevents substantial trigger movement such that the valve 114 cannot be opened.

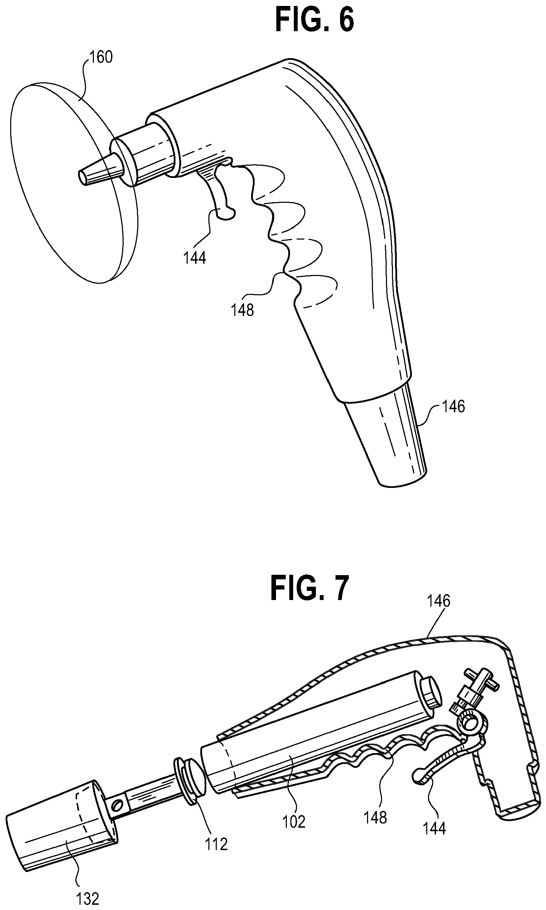

FIGS. 6-7 show an embodiment of a housing 146 of the wound irrigation device 100 along with certain other depicted features. Referring to FIG. 6, the housing 146 may include the ergonomic handle 148 located adjacent to the trigger 144, as discussed above. Further, the housing 146 may be coupled (e.g., fixed) to a splash guard 160, which may prevent the stream of irrigation fluid and contaminants from the wound from being splashed towards the operator of the wound irrigation device 100.

Referring to FIG. 7, the spring chamber 132 (or other actuation feature) may be removable from the housing 146, which may be advantageous where a specific pressure range may be selected at the site of a wound irrigation procedure, for example (e.g., where an operator can choose from different actuators having different characteristics). Optionally, the piston 112 also (or alternatively) may be removable from the barrel 102, which may allow the barrel 102 to be filled with an irrigation fluid by an operator, allowing for selection of a particular irrigation fluid, re-use after exhaustion of the fluid, etc.

FIG. 7 is an illustration of the wound irrigation device 100 in a partiallyassembled state with the housing 146 from FIGS. 5-6. Like certain embodiments described above, the wound irrigation device 100 of FIG. 7 includes an actuation spring 128 that is mechanically coupled to the piston 112, and the piston 112 is movable within the fluid chamber 104. In this embodiment, the spring chamber 132 includes a cap 170 that attaches to the housing 146 (e.g., via threads or another suitable attachment device). The cap 170 may be removable such that the actuation spring 128 may be placed in the spring chamber 132 by an operator, for example (which may allow selection of a particular spring with a particular spring constant and/or size). Further, the spring chamber 132 is partially defined by the barrel 102. That is, the cylinder defining the barrel 102 in this embodiment is segmented into two chambers, (1) the fluid chamber 104, and (2) a portion of the spring chamber 132. The piston 112, which is mechanically coupled to the actuation spring 128 via direct contact, separates the two chambers.

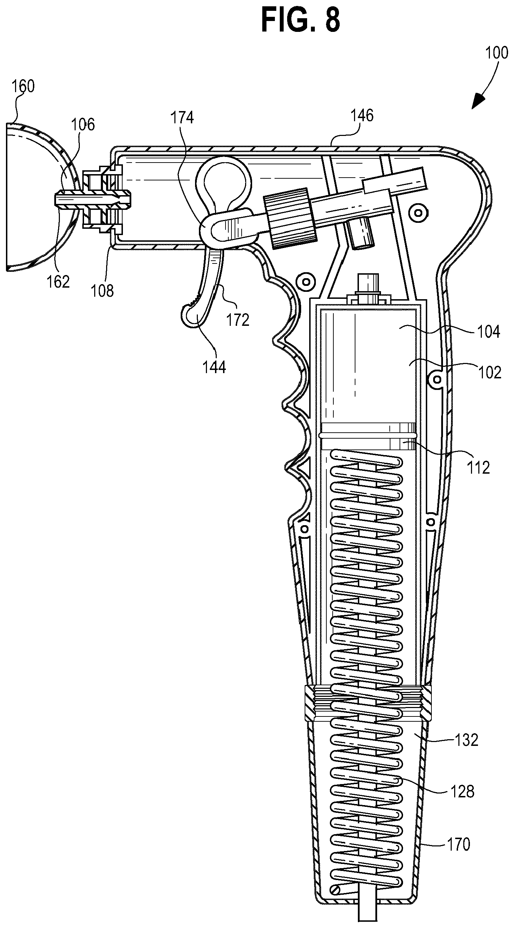

The trigger 144 of FIG. 8, which may be coupled to a valve within a fluid path between the fluid chamber 104 and the irrigation outlet 106 (as discussed above), may generally include an actuation portion 172 and a base portion 174. The actuation portion 172, which includes an extension for contact by a finger, may be pivotally connected to a base portion 174, and the base portion 174 may be configured to open and close the valve upon relative movement between the actuation portion 172 and the base portion 174. Optionally, the trigger 144 may include a leaf spring (not shown) and/or other element that creates a default tendency for the trigger 144 to move into a non-actuated state (e.g., where the valve is closed). Other trigger mechanics (and/or other actuators altogether) are also contemplated.

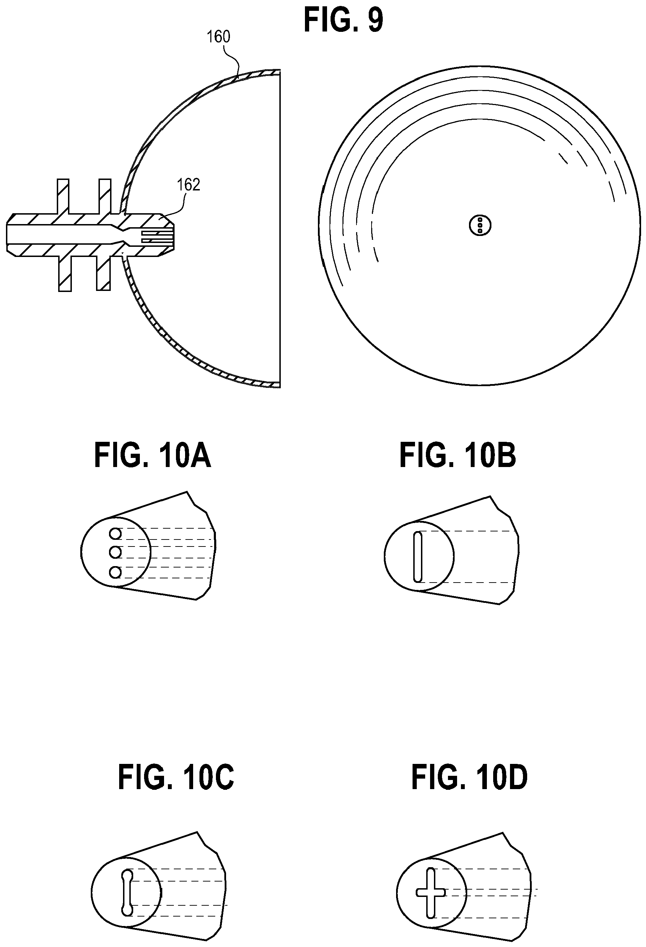

As discussed above, the irrigation outlet 106 of the wound irrigation device 100 may include at least one nozzle 162 located within the concave region of a splash guard 160. Optionally, the nozzle 162 may be modular relative to the housing 146 (e.g., capable of being connected and disconnected from the housing 146 (FIG. 8) by an operator). FIG. 9 shows one example of a nozzle 162 that may be coupled to the distal end 108 of the housing 146 shown in FIG. 8 (where the nozzle 162 is assembled or fixed to an optional splash guard 160). Advantageously, prior to initiation of an irrigation procedure, a medical professional and/or another operator may select and then connect a particular nozzle (e.g., from a set of nozzles) that is designed for a particular wound type, a particular pressure range, a particular patient or operator preference, etc. Alternatively, the nozzle 162 may be irremovable during normal use, and in some embodiments it may be formed as a unitary body with a portion of the housing 146 and/or another component (e.g., the splash guard 160). Notably, the above-mentioned flow path, valve, and nozzle are not depicted in FIGS. 6-8 (to facilitate viewing other features).

When a nozzle is included, it may be configured to provide any suitable stream shape. For example, a substantially-planar stream may be used, which may advantageously balance the need for precise control and aim of the stream while also providing relatively large stream coverage over a target area for time efficiency. For example, FIGS. 10A-D show four potential nozzle designs (which may be included with a modular nozzle set, for example), each providing a different (substantially) planar spray pattern that may be advantageous for certain wound irrigation procedures. In particular, the nozzle design in FIG. 10A includes multiple circular outlets arranged linearly on the distal tip of the nozzle such that multiple jets of irrigation fluid are discharged, forming an approximately planar stream. The nozzle design in FIG. 10B may provide a similar planar discharge stream, but without delineated jets. The nozzle designs in FIG. 10C and FIG. 10D are similar to the nozzle design in FIG. 10B, but additionally include regions where the discharge stream of irrigating fluid is enlarged (in the direction perpendicular to the main plane of the discharge stream) relative to other regions. Any other suitable stream type, whether planar or not, is contemplated.

While various embodiments of the present disclosure have been described, the present disclosure is not to be restricted except in light of the attached claims and their equivalents. One skilled in the relevant art will recognize that numerous variations and modifications may be made to the embodiments described above without departing from the scope of the present invention, as defined by the appended claims. Moreover, the advantages described herein are not necessarily the only advantages of the present disclosure and it is not necessarily expected that every embodiment of the present disclosure will achieve all of the advantages described.

## Claims

1. A wound irrigation device comprising:
a barrel having a fluid chamber at least partially filled with an irrigation fluid;
a piston located in the barrel, wherein the piston is movable between a terminal proximal position and a terminal distal position within the barrel to control a volume of the fluid chamber of the barrel; and
an actuation spring that is mechanically coupled to the piston, and
wherein the actuation spring is configured such that a pressure of the irrigation fluid within the fluid chamber continuously exceeds about 4 psi as the piston moves between the terminal proximal position and the terminal distal position.

2. The wound irrigation device of claim 1, further comprising an irrigation outlet and a valve, wherein the valve controls fluid communication between the irrigation outlet and the fluid chamber of the barrel.

3. The wound irrigation device of claim 2, wherein the valve is mechanically coupled to a trigger that extends outside of a housing of the wound irrigation device.

4. The wound irrigation device of claim 2, wherein the irrigation outlet includes at least one nozzle, the nozzle configured to form a planar stream.

5. The wound irrigation device of claim 1, wherein in a locked state, the piston is fixed in the terminal distal position and the pressure of the irrigation fluid within the fluid chamber is less than about 4 psi.

6. The wound irrigation device of claim 5, further comprising a locking device configured to selectively engage the piston such that, when the wound irrigation device is in the locked state, the piston is fixed relative to the barrel.

7. The wound irrigation device of claim 1, wherein the actuation spring includes at least one of a compression spring and an extension spring.

8. The wound irrigation device of claim 1, wherein the actuation spring contacts an anchor that is movable within a spring chamber, the anchor being fixed relative to the piston.

9. The wound irrigation device of claim 1, wherein the actuation spring directly contacts the piston.

10. The wound irrigation device of claim 1, further comprising a housing that at least partially surrounds the barrel, wherein the housing forms an ergonomic handle.

11. A wound irrigation device, comprising:
a barrel having a fluid chamber configured to be at least partially filled with an irrigation fluid;
a piston located in the barrel, wherein the piston is movable within the barrel to control a volume of the fluid chamber of the barrel; and
an actuation spring mechanically coupled to the piston, wherein during an operational state, the actuation spring applies a distal actuation force on the piston to pressurize the irrigation fluid within the fluid chamber.

12. The wound irrigation device of claim 11, further comprising an irrigation outlet and a valve, wherein the valve controls fluid communication between the irrigation outlet and the fluid chamber of the barrel.

13. The wound irrigation device of claim 12, wherein the valve is mechanically coupled to a trigger that extends outside of a housing of the wound irrigation device.

14. The wound irrigation device of claim 11, wherein in a locked state, the piston is fixed in a terminal distal position and a pressure of the irrigation fluid within the fluid chamber is less than about 4 psi.

15. The wound irrigation device of claim 14, further comprising a locking device configured to selectively engage the piston such that, when the wound irrigation device is in the locked state, the piston is fixed relative to the barrel.

16. A wound irrigation device, comprising:
a barrel having a fluid chamber;
a piston located in the barrel, wherein the piston is movable within the barrel to control a volume of the fluid chamber of the barrel; and
wherein the wound irrigation device comprises a locked state and an operational state,
wherein in the locked state, the piston is fixed relative to the barrel, and
wherein in the operational state, an actuation force applied to the piston in the distal direction pressurizes an irrigation fluid within the fluid chamber.

17. The wound irrigation device of claim 16, further comprising a locking device configured to selectively engage the piston such that, when the wound irrigation device is in the locked state, the piston is fixed relative to the barrel.

18. The wound irrigation device of claim 16, further comprising an irrigation outlet and a valve, wherein the valve controls fluid communication between the irrigation outlet and the fluid chamber of the barrel.

19. The wound irrigation device of claim 18, wherein the valve is mechanically coupled to a trigger that extends outside of a housing of the wound irrigation device.

20. The wound irrigation device of claim 16, wherein in the locked state, the piston is fixed in a terminal distal position and a pressure of the irrigation fluid within the fluid chamber is less than about 4 psi.
